# EUROPEAN PATENT APPLICATION

(11) **EP 0 804 914 A1**
(43) Date of publication of application: **05.11.1997**
(21) Application number: 96106722.0
(22) Date of filing: 29.04.1996
(51) Int. Cl.: A61F 13/15

(54) **Disposable absorbent article capable of self-shaping in use**

(71) Applicant: THE PROCTER & GAMBLE COMPANY, Cincinnati, Ohio 45202 (US)
(72) Inventor: Ciammaichella, Fabio, 65129 Pescara (IT); Corzani, Italo, 66100 Chieti (IT)
(74) Representative: Canonici, Jean-Jacques

(57) **Abstract**

A disposable absorbent article for wearing adjacent a body discharge area having a longitudinal centreline and a lateral centreline orthogonal thereto and defining longitudinal and lateral directions respectively. The disposable absorbent article comprises a liquid pervious topsheet, a backsheet joined to the topsheet and an absorbent core intermediate the backsheet and the topsheet; the article is substantially flat prior to use. The disposable absorbent article further comprises means for inducing forming or bending of the article into a tridimensional structure while being worn by a user. The means are activated by body fluids or body heat.

## Description

### FIELD OF THE INVENTION

The present invention relates to disposable absorbent articles. Disposable absorbent articles are considered to be absorbent devices designed to be worn externally of the body by a user and to receive fluids discharged from the body. In particular the present invention relates to substantially flat disposable absorbent sanitary napkins, catamenials, incontinence inserts, pantiliners and diapers comprising means for inducing forming or bending of the article into a tridimensional structure while being worn by a user. The means is activated by body fluids or body heat and provides the article with a self-shaping capability during the use.

### BACKGROUND OF THE INVENTION

In their basic form, disposable absorbent articles comprise an absorbent core interposed between a pervious body-contacting element (alternatively referred to as a topsheet or an overwrap) and an impervious protective barrier (alternatively referred to as a backsheet). The absorbent element is, of course, intended to receive and contain the fluids discharged from the body. The body-contacting element is intended to provide comfortable and dry-feeling contact with body surfaces while allowing free passage of fluids therethrough into the absorbent element. The protective barrier is intended to prevent the fluids which are expelled or which escape from the absorbent element from soiling the user's garments.

In addition to the three functional elements mentioned above, disposable absorbent articles are generally provided with means for supporting the device adjacent the user's crotch area, even as the user moves, where it can most effectively perform its intended function. Typically, absorbent articles as sanitary napkins are provided with an adhesive attachment means for securing the device to the inner crotch area of the user's undergarments.

Improved fluid interception can occur if the absorbent article is in close contact with the body of the wearer.

While previously known absorbent articles perform their intended function, each conventional design can be further improved in one or more of absorbency of body fluids, protection of the user's garments from soiling, and/or physical comfort to the user.

With respect to disposable sanitary napkins several attempts have been made in the art to improve body contact with the wearer, and hence absorb fluids upon discharge and thereby minimize soiling by providing a sanitary napkin having an anatomically shaped configuration, particularly including those that are raised upwardly or humped in their medial portions so as to be near or in contact with the pudendal region when worn.

On female users these sanitary napkins attempt to contact and absorb menses immediately as it leaves the vestibule.

U.S. Patent No. 5,300,055 describes a sanitary napkin provided with a flexure-resistant deformation element. This deformation element is capable of forming and maintaining a substantially convex upward configuration when the article is worn, upon lateral compressive forces exerted by the user's thighs: this is achieved since the deformation element is capable of bending in a predetermined and desired way along one or more longitudinal flexure hinges in order to assume the upward convex configuration during wear. The preferred configurations comprise W-shaped or inverted V-shaped cross-sections for at least a central region of the sanitary napkin; alternatively, the napkin may have a cup-shaped front region and a convex upward configuration in the back region. While this embodiment is capable of forming an effective tridimensional convex upward configuration when the article is worn, it may still have the disadvantage that this configuration is achieved in substantially only one predetermined way, according to the pattern of flexure hinges impressed in the deformation element. Moreover, the deformation element constitutes a well defined structure comprised in the napkin and may be relatively stiff and cumbersome.

Another attempt to impart a tridimensional configuration to a sanitary napkin is disclosed in PCT application published as WO 95/17148. The application describes a sanitary napkin in which a convex upward configuration is achieved by means of at least an elastic element preferably positioned under the absorbent core and joined to it; the elastic element spans the longitudinal centreline of the absorbent core and upon contraction is capable of shortening the sanitary napkin in the lateral direction, so creating the desired configuration. Longitudinal lines of weakness may be provided in the sanitary napkin in order to facilitate the bending of the napkin in the desired way. While this type of structure is capable of staying in closer contact with the body of the user, it may have the disadvantage of a poor conformability to the various anatomies; in an attempt to solve this problem a more complex configuration is described in which a tailored fit to the anatomy is provided by means of various elastic elements with different contracting forces applied at different portions of the absorbent core.

In any case the structures described above have a pre-formed configuration which is designed and achieved prior to use; moreover the tridimensional sanitary napkin is more difficult to be processed and packaged than a conventional flat pad.

### SUMMARY OF THE INVENTION

The present invention relates to disposable absorbent articles for wearing adjacent a body discharge area, which are substantially flat prior to use. The substantially flat disposable absorbent article has a longitudinal centreline and a lateral centreline orthogonal thereto that define longitudinal and lateral directions respectively. The disposable absorbent article comprises a liquid pervious topsheet, a backsheet, preferably liquid impervious, joined to said topsheet, and an absorbent core intermediate the topsheet and the backsheet. The term "substantially flat", as used herein, refers to articles that have their main extension in one plane in contrast to being shaped. The article further comprises means for inducing forming or bending of the article into a tridimensional structure while being worn by a user, wherein the means is activated by body fluids or body heat.

### BRIEF DESCRIPTION OF THE DRAWINGS

While the specification concludes with claims particularly pointing out and distinctly claiming the present invention, it is believed that the present invention will be better understood from the following description in conjunction with the following drawings:
FIG. 1 is a top plan view of one embodiment of a sanitary napkin according to the present invention;
FIG. 2 is a cross-sectional view of the sanitary napkin shown in FIG. 1 as taken along section line 2-2;
FIG. 3 is a cross-sectional view similar to that of FIG. 2, showing the means for inducing bending of the sanitary napkin after activation during wear;
FIG. 4 is a cross-sectional view of another embodiment of a sanitary napkin according to the present invention;
FIG. 5 is a cross-sectional view similar to that of FIG. 4, showing the means for inducing bending of the sanitary napkin after activation during wear.

### DETAILED DESCRIPTION OF THE INVENTION

This invention relates to a disposable absorbent article which exhibits absorbency for bodily fluids, the protection of the user's garments from soiling, improved physical comfort to the user, and which is easy to produce and to package. The disposable absorbent article is described below by reference to a sanitary napkin or catamenial. The term "sanitary napkin", as used herein, refers to an article which is worn by females adjacent to the pudendal region and which is intended to absorb and contain the various fluids which are discharged from the body (e.g., vaginal discharges, menses, and/or urine) and which is intended to be discarded after a single use. The disposable absorbent article is substantially flat prior to use.

The term "substantially flat", as used herein, refers to articles that have their main extension in one plane in contrast to being shaped. In a preferred embodiment a substantially flat article will have an absorbent core of constant thickness or, at least, will have an absorbent core that is not shaped in a direction which is orthogonal to the absorbent core itself. This does not exclude a general curvature of the absorbent core. It will be apparent to the man skilled in the art to which extent products can deviate from absolute flat shape and still benefit from the during the use shaping according to the present invention.

Sanitary napkins with longitudinal side cuffs, which may be optionally elasticated, and sanitary napkins with a moderate curvature are therefore within the scope of the present invention, provided that their absorbent core is not shaped prior to use in a direction that is orthogonal to the absorbent core itself.

The term "use", as used herein, refers to the period of time that starts when the absorbent article is actually put in contact with the anatomy of the user.

The terms "joined" or "affixed", as used herein, encompasses configurations whereby a first member is directly connected to a second member and configurations whereby a first member is indirectly connected to a second member by connecting the first member to intermediate members which in turn are connected to the second member.

Interlabial devices which reside partially within and partially external of the wearer's vestibule are also within the scope of this invention. As used herein, the term "pudendal" refers to the externally visible female genitalia and is limited to the labia majora, the labia minora, the clitoris, and the vestibule.

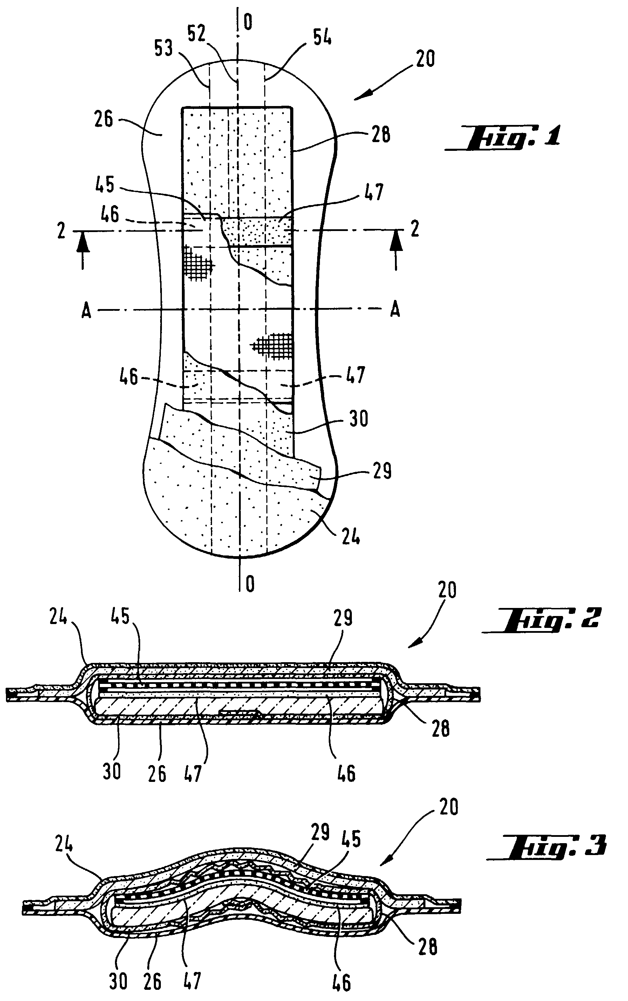

In FIGS. 1 and 2, one preferred embodiment of a sanitary napkin 20 of the present invention is shown. Fig. 1 is a plan view of the sanitary napkin 20 of the present invention in its flat-out state prior to use with portions of the structure being cut-away to more clearly show the construction of the sanitary napkin 20 and with the portion of the sanitary napkin 20 which faces or contacts the wearer oriented towards the viewer. As shown in FIGS. 1 and 2, the sanitary napkin 20 preferably comprises a liquid pervious topsheet 24, a liquid impervious backsheet 26 joined to the topsheet 24, an absorbent core 28 intermediate the topsheet 24 and the backsheet 26, and an acquisition layer 29 (sometimes referred to as a secondary topsheet) comprised between the topsheet 24 and the absorbent core 28.

Referring to FIGS. 1 and 2, plural lines of weakness 52, 53, and 54 may be disposed in the core 28. Inside the core 28 and joined to it through a plastic net 45 are means 46 for inducing forming or bending of the sanitary napkin 20 into a tridimensional structure during wear.

The sanitary napkin 20 has two surfaces, a body contacting or facing surface, and a garment facing or contacting surface. The absorbent core 28 has corresponding body facing and garment facing surfaces. The sanitary napkin 20 has two centrelines, a longitudinal centreline O-O and a transverse centreline A-A orthogonal thereto. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral", as used herein, are interchangeable, and refer to a line, axis, or direction which lies within the plane of the sanitary napkin 20 and is generally perpendicular to the longitudinal direction. The Z-direction is orthogonal to both the longitudinal and lateral directions of the sanitary napkin 20 and extends outwardly from the plane of the sanitary napkin 20, which is defined by the longitudinal centreline O-O and the lateral centreline A-A. The term "longitudinally oriented" refers to a direction ±45 degrees of the longitudinal direction; the term "laterally oriented" refers similarly to any other direction in the plane of the sanitary napkin 20.

The long edges of the sanitary napkin 20, which are aligned with the longitudinal centreline O-O, are the longitudinal side margins of the sanitary napkin 20. The ends of the sanitary napkin 20 joining the longitudinal side margins are the transverse ends of the sanitary napkin 20. Collectively the longitudinal side margins and transverse ends of the sanitary napkin 20 define its periphery. Similarly, the absorbent core 28 of the sanitary napkin 20 has a periphery defined by alternatively disposed longitudinal side margins and transverse ends.

Tridimensional structures of the sanitary napkin 20 are those in which the sanitary napkin structure is caused to extend, at least partially, in the Z-direction, for example by means of bending, in order to more closely conform to the user's anatomy. Said expansion takes place in a direction that goes from the garment facing surface towards the body facing surface of the sanitary napkin 20. Suitable tridimensional structures may comprise concave upward configurations with or without side cuffs. Particularly preferred are tridimensional structures with a convex upward configuration that are inclusive of, but not limited to, W-shapes and inverted U-shapes or inverted V-shapes. A W-shaped configuration is generally preferred due to its longitudinal dams adjacent and inboard of the longitudinal side margins. Additionally, in a W-shaped configuration, the cross-sectional contour of the central portion of the sanitary napkin more closely matches the labia of the typical wearer.

The topsheet 24 is compliant, soft feeling, and non-irritating to the wearer's skin. Further, the topsheet 24 is liquid pervious, permitting liquid to readily penetrate through its thickness. A suitable topsheet 24 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibres (e.g., wood or cotton fibers), synthetic fibres (e.g., polymeric fibres such as polyester, polypropylene, or polyethylene fibres); or from a combination of natural and synthetic fibres.

A preferred topsheet comprises an apertured formed film. Apertured formed films are preferred for the topsheet because they are pervious to body fluids and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer.

Suitable formed films are described in U.S. Pat. No. 3,929,135, issued to Thompson on December 30, 1975; U.S. Pat. No. 4,324,246, issued to Mullane, et al. on April 13, 1982; U.S. Pat. No. 4,342,314, issued to Radel, et al. on August 3, 1982; U.S. Pat. No. 4,463,045, issued to Ahr, et al. on July 31, 1984; and U.S. Pat. No. 5,006,394, issued to Baird on April 9, 1991. A preferred topsheet for the absorbent article of the present invention is a formed film described in one or more of the above patents and marketed on sanitary napkins by The Procter & Gamble Company of Cincinnati, Ohio as "DRI-WEAVE".

In a preferred embodiment of the present invention, the body or exposed surface of the formed film topsheet is hydrophilic so as to help liquid transfer through the topsheet faster than if the body surface were not hydrophilic so as to diminish the likelihood that menstrual fluid will flow off the topsheet rather than flowing into and being absorbed by the absorbent core.

The sanitary napkin 20 may comprise an acquisition layer 29 positioned between the topsheet 24 and the absorbent core 28. The acquisition layer 29 may serve several functions including improving wicking of body fluids over and into the absorbent core 28. By improving the wicking of body fluids, the acquisition layer 29 provides a more even distribution of the body fluids throughout the absorbent core 28. The acquisition layer 29 may be comprised of several different materials including nonwoven or woven webs of synthetic fibres including polyester, polypropylene, or polyethylene; natural fibres including cotton or cellulose; blends of such fibres; or any equivalent materials or combinations of materials. Examples of sanitary napkins having an acquisition layer and a topsheet are more fully described in U.S. Pat. No. 4,950,264 issued to Osborn and U.S. Pat. Application Serial No. 07/810,774, "Absorbent Article Having Fused Layers", filed December 17, 1991 in the names of Cree, et al.

The topsheet 24, the acquisition layer 29 and the absorbent core 28 may be associated in any suitable manner. Suitable manners include, but are not limited to, associating the topsheet 24, the acquisition layer 29 and the absorbent core 28 with adhesives such as by spray-gluing or applying lines or spots of adhesive between the topsheet 24 and the acquisition layer 29 and/or between the acquisition layer 29 and the absorbent core 28.

Alternatively, or additionally, the association between the layers may be achieved by fibre entanglement or by a plurality of discrete fusion bonds.

Particularly, to insure proper fluid transfer between the topsheet 24 and the acquisition layer 29 it is preferred that the topsheet 24 be substantially continuously secured to the underlying acquisition layer 29 throughout their common interface. By substantially continuously securing the topsheet 24 to the underlying acquisition layer 29 the topsheet 24 will have a reduced tendency to separate from the acquisition layer 29 during use. Separation of the acquisition layer 29 from the topsheet 24 may inhibit fluid transfer from the topsheet 24 into the underlying acquisition layer 29 and therefore into the absorbent core 28.

The absorbent core 28 may be any absorbent means which is generally compressible, conformable, resilient, non-irritating to the wearer's skin and capable of absorbing and containing body fluids. The absorbent core 28 may be manufactured from a wide variety of liquid absorbent materials commonly used in disposable sanitary napkins, and other disposable absorbent articles. Examples of suitable absorbent materials include comminuted wood pulp (which is generally referred to as airfelt), creped cellulose wadding, modified cross-linked cellulose fibres (such as those described in U.S. Patent No. 5,217,445 issued to Young, et al. on June 8, 1993), capillary channel fibres (that is, fibres having intra-fibre capillary channels such as those described in U.S. Patent No. 5,200,248 issued to Thompson, et al. on April 6, 1993), absorbent foams (such as those described in U.S. Patent No. 5,260,345, issued to DesMarais, et al. on November 9, 1993 and U.S. Patent No. 5,268,244 issued to DesMarais, et al. on December 7, 1993), thermally bonded airlaid materials (such as those material described in U.S. Patent Application Serial No. 08/141,156, entitled "Catamenial Absorbent Structures Having Thermally Bonded Layers For Improved Handling of Menstrual Fluids and Their Use In Catamenial Pads Having Improved Fit and Comfort" filed in the name of Richards, et al. on October 21, 1993 (P&G Case 5051)), absorbent sponges, synthetic staple fibres, polymeric fibres, hydrogel-forming polymer gelling agents, peat moss, or any equivalent materials or combinations of materials. Suitable absorbent cores comprising foams are described in European Applications 0 598 833, 0 598 823 and 0 598 834.

The absorbent capacity of the absorbent core 28 should be compatible with the intended body fluid loading for the sanitary napkin 20. Further, the absorbent capacity of the absorbent core 28 may be varied to accommodate wearers ranging in the expected amount of body fluid volume. For instance, a different absorbent capacity may be utilized for sanitary napkins intended for day time use as compared with those intended for night time use, or for sanitary napkins intended for use by teenage females as compared with those intended by more mature women.

Materials selected for use as the absorbent core 28 are preferably compliant, soft, comfortable, compressible and resilient to enhance body fit and comfort of the absorbent core 28. Preferably, the absorbent core 28 is compressible such that it will deform under relatively small forces that are experienced during normal use. In addition to being compressible, the materials of the absorbent core 28 need to be flexible and conformable such that the absorbent core 28 is able to provide improved fit into and around the labia and perineum when the tridimensional structure is formed during the wearing time. The ability to follow the topography of the anatomy will provide intimate contact with the exposed genitalia of the female user. Intimate contact with the exposed female genitalia helps provide better fluid transfer from the user into the absorbent core 28. While these characteristics of the absorbent core 28 allow for improved fit, they also cause the product to be both soft and comfortable for the wearer.

In the embodiment illustrated in FIGS. 1 to 3, the absorbent core 28 is comprised of an absorbent layer 30 made of a thermally bonded airlaid material longitudinally folded twice on itself and comprising particles of absorbent gelling material therebetween, which are not shown for clarity.

The absorbent core 28 further comprises means 46 for inducing forming or bending of the sanitary napkin 20 into the desired tridimensional structure while the sanitary napkin 20 is being worn. In a preferred embodiment the bending and final shaping of the sanitary napkin 20 into the tridimensional structure is provided by the contraction or shrinking of the material that constitutes the means 46 and that is activated during wear by body fluids or body heat.

The contracting material that constitutes the means 46 may be in form of film, stripes, yarns, fabric, or any other form, provided that this does not interfere with the contraction or shrinking itself, which typically occurs in the transverse direction in one preferred embodiment of the sanitary napkin 20 illustrated in FIGS. 1 to 3, and provided that the physical characteristics and the form of the material are capable of imparting a contractive force sufficient to induce formation of the desired tridimensional structure.

Suitable shrinkage values may vary between 5% and 60%, preferably between 30% and 40%.

Suitable materials may be those that shrink upon contact with water or water-containing body fluids. Composite materials may also be used, which comprise an elastic material laminated in stretched condition to a non elastic, stiff material that dissolves or weakens upon contact with body fluids, so releasing the elastic tension of the elastic material that therefore is allowed to contract and to impart the desired tridimensional structure to the article during wear.

As an alternative, materials that contract or shrink upon activation by body heat may also be used. Heat-shrinkable materials that contract at temperatures comprised in the range of 28 to 37 °C, that is in the range of temperatures that can be found in close contact with the human body, may be suitably used for the means 46. Heat activatable laminated structures which are similar to those described above may make use, as the stiff material to be laminated to the elastic material in the stretched condition, of materials that have a glass transition temperature comprised in the range defined above; at this temperature they change their state from hard and rigid to soft and deformable, so releasing the elastic tension of the associated elastic material during wear of the absorbent article.
Referring again to FIGS. 1 to 3, the absorbent core 28 may comprise a stiffening element 45; the stiffening element 45 is a flexible, liquid pervious material which is more rigid than the structure of the sanitary napkin 20 and helps it achieve, in use, the desired tridimensional structure. It has been found that preferred stiffness values for the stiffening element 45 may range from 0.002 N to 0.15 N, more preferably from 0.005 N to 0.08 N, as measured according to the standard test method ISO 2493-1992. In a preferred embodiment illustrated in FIGS. 1 to 3 the stiffening element comprises a plastic net 45, for example a polypropylene net having a stiffness of about 0.01 N, joined to the inner surface of the layer 30 opposite to the garment facing surface of the absorbent core 28, preferably along the longitudinal side margins; in the embodiment illustrated in FIG. 1 the plastic net 45 has about the same width as the absorbent core 28 and is shorter than it, being about one third of the total length of the absorbent core 28; the plastic net 45 is centered both on the transverse centreline A-A and on the longitudinal centreline O-O. The absorbent core 28 further comprises means 46 for inducing forming or bending of the sanitary napkin 20 into the desired tridimensional structure while the sanitary napkin 20 is being worn. Means 46 comprises two rectangular elements 47 of a material that is capable of shrinking upon activation by body fluids; each element 47 spans the longitudinal centreline O-O of the sanitary napkin 20 and is generally parallel to the transverse centreline A-A. The length of each element 47 generally corresponds to the width of the plastic net 45, while the width in longitudinal direction is usually smaller. In one preferred embodiment illustrated in FIGS. 1 to 3 each element 47 is about 1 cm wide, while the lengths of the absorbent core 28 and of the plastic net 45 are about 22 cm and about 7 cm, respectively; the width of the absorbent core is about 6 cm, to which the common transverse dimensions of the plastic net 45 and of each element 47 roughly correspond.

Each element 47 is joined in any suitable manner, for example by stitching or gluing, to the lower surface of the plastic net 45 preferably at its transverse ends corresponding to the longitudinal side margins of the plastic net 45.

One suitable material for element 47 may be a polyvinyl alcohol composition oriented and preferably crosslinked so as to shrink without significant solubility upon contact with body fluids; particularly preferred is the composition produced and marketed by Nitivy Co., Ltd. under the tradename of SOLVRON. SOLVRON is available in form of yarns, continuous filaments, staple fibres or fabric. Useful fabrics may comprise 100% SOLVRON fibres or any mixtures of SOLVRON fibres with other synthetic or natural fibres, for example cotton, nylon, polyethylene therephthalate or polypropylene fibres. A suitable fabric, sold under the tradename of GFX 5788, is made of 100% SX grade, 56 denier SOLVRON fibre; upon contact with body fluids the fabric shows a shrinkage of about 35% both in longitudinal and in transverse directions.

Sanitary napkin 20 may also comprise one or more longitudinally oriented weakness lines. As illustrated in FIG. 1 the sanitary napkin 20 comprises longitudinally oriented weakness lines 52, 53, and 54, impressed for example by embossing the absorbent core 28. Line of weakness 52 preferably runs along the longitudinal centreline O-O of the sanitary napkin 20, while lines of weakness 53 and 54 are preferably symmetrically disposed about the longitudinal centreline O-O; lines of weakness may be generally rectilinear and parallel to one another, as illustrated in FIG. 1, or may follow any other suitable pattern in order to assist the sanitary napkin 20 to assume the desired tridimensional structure. Lines of weakness may be formed by embossing the core 28 and may optionally further be embossed through the topsheet 24 and/or the acquisition layer 29.

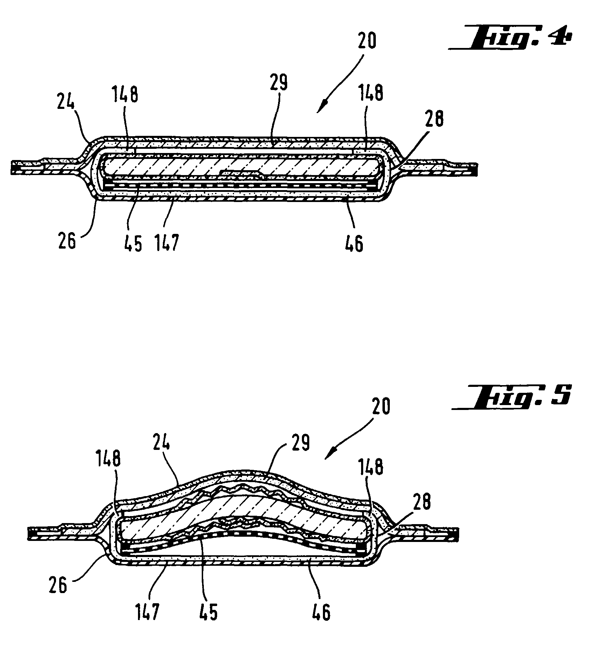

The sanitary napkin 20 is produced and packaged as a conventional flat product, as illustrated in FIGS. 1 and 2. After the sanitary napkin 20 has been worn, as soon as the absorbed body fluids come in contact with the material of one of the elements 47, it will begin to shrink in both directions; since the dimension of each element 47 which is parallel to the longitudinal centreline O-O is relatively small, as compared to the dimension of each element 47 which is parallel to the transverse centreline A-A, the overall effect of the shrinkage of each element 47 in longitudinal direction may be negligible. FIG. 3 shows the effect of the shrinking of an element 47 in transverse direction. As the element 47 shrinks it shortens the absorbent core 28 through the plastic net 45, and therefore the sanitary napkin 20 as well, in transverse direction. The plastic net 45, being stiffer than the structure of the sanitary napkin 20, helps to push the overlaying layers of the absorbent core 28, acquisition layer 29 and topsheet 24 towards the anatomy of the user under the action of the shrinking element 47. The absorbent core 28 is induced to bend along the weakness lines 52, 53, and 54 thus forming the inverted V-shaped tridimensional structure shown in FIG. 3. As it is known in the art, in order to assist the absorbent core 28 to bend in the desired way, the embossing may be made on the side in which the bending is expected to take place. In the embodiment of FIG. 3 the central line of weakness 52 is preferably embossed on the garment facing surface of the absorbent core 28, while the side weakness lines 53 and 54 are preferably embossed on the body facing surface of the absorbent core. Lines of weakness may be achieved with any other means known in the art, other than embossing.

The shrinking of the material forming each element 47 takes place only upon activation by the absorbed fluid, that is only during the use of the sanitary napkin 20 and in close contact with the user's anatomy; the formation of the tridimensional structure can therefore achieve a much better fit with the anatomy of the user. Moreover, the different elements 47 may start to shrink at different times, as soon as each one is reached by the fluid; the formation of the tridimensional structure may also fit, therefore, the different possible ways in which the body fluids may be released by various users.

The sanitary napkin of the present invention is flat prior to use, and may be therefore manufactured and packaged more easily than a conventional elasticated or pre-formed article. Since the tridimensional structure is formed only during the use, the sanitary napkin of the present invention is also easier to wear.

In another preferred embodiment of the present invention a fabric that comprises 25% of the same SOLVRON fibre as described above and 75% of nylon fibres may be used; if polyvinyl alcohol fibres constitute only at least part of the weft of the fabric, while the warp is comprised of nylon alone, the fabric will shrink in the direction of the weft only upon activation by body fluids. The fabric may be accordingly disposed in the sanitary napkin being oriented so as to provide contraction along the transverse direction only. A mixed fabric is moreover easier to bond to a substrate, e.g. by known adhesive means or by thermobonding, than a 100% polyvinyl alcohol composition.

In an alternative embodiment of the present invention, the sanitary napkin 20 may have two flaps (not shown), each of which are adjacent to and extend laterally from the side edge of the absorbent core. The flaps are configured to drape over the edges of the wearer's panties in the crotch region so that the flaps are disposed between the edges of the wearer's panties and the wearer's thighs. The flaps serve at least two purposes. First, the flaps help serve to prevent soiling of the wearer's body and panties by menstrual fluid, preferably by forming a double wall barrier along the edges of the panty. Second, the flaps are preferably provided with attachment means on their garment facing surface so that the flaps can be folded back under the panty and attached to the garment facing side of the panty. In this way, the flaps serve to keep the sanitary napkin properly positioned in the panty.

The flaps may be constructed of various materials including materials used for the topsheet 24, backsheet 26, combinations thereof, and may be a laminate having tissue in the centre. Further, the flaps may be a separate element attached to the main body of the sanitary napkin 20 or can comprise extensions of the topsheet 24 and/or backsheet 26. It is recommended, however, that the flaps have a liquid impervious backsheet to prevent body fluids which reach the flaps from soiling the edges of the wearer's panties.

Preferred flaps that are suitable or adaptable to the sanitary napkin 20 of the present invention are disclosed in U.S. Pat. No. 4,687,478 issued to Van Tilburg on Aug. 18, 1987; U.S. Pat. No. 4,589,876 issued to Van Tilburg on May 20, 1986; and U.S. Pat No. 4,608,047 issued to Mattingly on Aug. 26, 1986.

Optionally, the sanitary napkin 20 may comprise components that naturally wrap the sides of a wearer's panties. Sanitary napkins having components that naturally wrap the sides of a wearer's panties suitable for use with the sanitary napkin 20 of the present invention are disclosed in U.S. Patent Application Serial No. 08/096,121 entitled "Absorbent Article having Panty Covering Components that Naturally Wrap the Sides of Panties", filed July 22, 1993, in the names of Lavash, et al and U.S. Patent Application Serial No. 08/277733 entitled "Absorbent Articles Having Undergarment Covering Components with Zones of Extensibility", filed July 20, 1994, in the names of Weinberger, et al.

The means 46 for inducing forming or bending of the sanitary napkin 20 into a tridimensional structure during wear may be comprised in the sanitary napkin 20 in any other suitable position and/or orientation in order to get the desired tridimensional structure.

In an alternative embodiment illustrated in FIGS. 4 and 5 the means 46 are positioned beneath the absorbent core 28 in a sanitary napkin 20. With reference to FIG. 4, the plastic net 45 has the same shape and dimensions as that illustrated in FIG. 1, but it is positioned underneath the absorbent core 28, and is joined to it preferably along both longitudinal side margins. The means 46 comprises two elements 147 joined to the lower surface of the plastic net 45 along the longitudinal side margins of the plastic net 45; each element 147 has a transverse dimension that is longer than the common transverse dimension of the absorbent core 28 and of the plastic net 45, and the two free ends 148 of each element 147 are folded over the absorbent core 28. The sanitary napkin 20 is worn as a conventional flat article; as the body fluid is received by the acquisition layer 29 it diffuses through it and wicks over and into the absorbent core 28, and at the same time it reaches the free ends 148 of one element 147, so activating the shrinking of the material and therefore the bending of the sanitary napkin 20 into the preferred convex upward tridimensional structure, as illustrated in FIG. 5.

In another alternative embodiment, the shrinkable means for inducing the formation of the preferred tridimensional structure may comprise different materials placed in different positions of the sanitary napkin and optionally having different contraction properties in order to generate tridimensional structures that may be asymmetrical in longitudinal and/or in transverse direction. An example of an asymmetrical tridimensional structure is known in the so called labial sanitary napkins, in which a convex upward configuration is achieved in the rear portion of the sanitary napkin, which is in turn substantially concave in its front portion.

## Claims

1. A disposable absorbent article for wearing adjacent a body discharge area, said article having a longitudinal centreline and a lateral centreline orthogonal to said longitudinal centreline and defining longitudinal and lateral directions respectively, said article comprising a liquid pervious topsheet, a backsheet joined to said topsheet, an absorbent core intermediate said topsheet and said backsheet, said article being substantially flat prior to use, characterized in that said article further comprises means for inducing forming or bending of said article into a tridimensional structure while being worn by a user, said means being activated by body fluids or body heat.

2. A disposable absorbent article according to claim 1, characterized in that said means provides said article with a substantially convex upward configuration.

3. A disposable absorbent article according to any preceding claim, characterized in that said means is joined to said absorbent element at attachment positions disposed on opposite sides of said longitudinal centreline and extending laterally across, whereby said means upon activation shortens said disposable absorbent article substantially in lateral direction to provide said tridimensional structure.

4. A disposable absorbent article according to any preceding claim, characterized in that said means comprises a material which is capable of shrinking upon contact with body fluids.

5. A disposable absorbent article according to any preceding claim, characterized in that said means comprises a material which is capable of shrinking upon activation by body heat.

6. A disposable absorbent article according to claim 4, characterized in that said material comprises a water shrinkable polyvinyl alcohol composition.

7. A disposable absorbent article according to claim 6, characterized in that said material comprises water shrinkable polyvinyl alcohol fibres.

8. A disposable absorbent article according to any preceding claim, characterized in that it further comprises at least one longitudinally oriented line of weakness therein, preferably disposed along said longitudinal centreline.

9. A disposable absorbent article according to any preceding claim, characterized in that it further comprises a stiffening element joined to said absorbent element and to said means for inducing forming or bending of said articles into a tridimensional structure.

10. A disposable absorbent article according to any preceding claim, characterized in that it is a sanitary napkin or a pantiliner.
